# EUROPEAN PATENT SPECIFICATION

(11) **EP 2 544 641 B1**
(45) Date of publication and mention of the grant of the patent: **20.04.2016**
(21) Application number: 11715016.9
(22) Date of filing: 11.03.2011
(51) Int. Cl.: A61F 13/00, A61F 13/15

(54) **SACHET**
SÄCKCHEN
SACHET

(30) Priority: 11.03.2010 GB 201004001
(43) Date of publication of application: 16.01.2013
(73) Proprietor: Datesand Limited, Manchester, Lancashire M11 1XD (GB)
(72) Inventor: WOOD, Jonathon, Manchester Lancashire M11 1XD (GB)
(74) Representative: Wilson Gunn
(86) International application number: PCT/GB2011/050490
(87) International publication number: WO 2011/110868

(56) References cited:
- EP-A2- 0 294 189
- WO-A1-93/06802
- US-A- 4 291 697

## Description

The present invention relates to a sachet.

Sachets comprising a super absorbent polymer material are used for sanitary purposes in the hospital environment to clean up spillages, vomit and other liquid human waste material. Super absorbent material is useful because it can absorb and retain large quantities of liquid relative to its own mass so even a small amount is an effective cleaning tool.

Conventional sachets are formed from water permeable paper or water soluble synthetic polymer such as polyvinlyl alcohol and contain a small quantity of powdered super absorbent material. The paper sachets are designed to burst or rupture when exposed to water containing liquid as a result of the expansion of the superabsorbent material whose volume eventually exceeds the volume of the sachet. It is important that the sachet ruptures when exposed to liquid so that the superabsorbent material can continue to expand and is released and dispersed throughout the liquid. This maximises the effectiveness of the absorbent material and ensures that it absorbs as much water as possible. It is not a requirement that the polymer sachets rupture when exposed to water containing liquid because they will eventually dissolve and release the absorbent material.

Both types of sachet have advantages and disadvantages. Paper sachets can be produced at a higher rate (up to 100 times faster) than synthetic polymer sachets which tend to melt if they are run through manufacturing machines too quickly.

Consequently, paper sachets can be manufactured more efficiently and cheaply that synthetic polymer sachets. However, paper sachets do not have a 100% burst rate which means that some sachets fail to release the absorbent material when exposed to liquid, especially thick liquids such as vomit. Since synthetic polymer sachets are not dependent on upon bursting, they always release the absorbent material when exposed to water containing liquid and are not therefore subject to the same failure rate as paper sachets.

One proposed solution is to use a paper of lower tensile strength which is more likely to rupture when subjected to the forces exerted by swelled absorbent material. However, a problem with using such paper is that it cannot withstand the manufacturing process and tends to burn up or tear when run through manufacturing machines.

US 4,291,697 discloses a cleaning device for medical purposes, which comprises a sealable sponge portion which encapsulates a pressure rupturable pouch containing cleaning or antiseptic liquid. Applying pressure to the exterior of the sponge causes the pouch to rupture, allowing the liquid to spread outwards from the interior of the sponge to the sponge surface. Non-woven absorbent material may be sealed inside the sponge to absorb some of the liquid when it is released from the ruptured pouch.

An object of embodiments of the invention is to provide a sachet that is cheap and efficient to manufacture and has a high rupture rate.

According to an aspect of the present invention, there is provided a sachet defining a volume, the sachet comprising a sheet of material fastened to itself and/or another sheet of material and containing a quantity of dry granules of sodium polyacrylate capable of absorbently swelling to exceed the volume of the sachet when the sachet is exposed to liquid, wherein the fastening means comprises a watersoluble, heat activatable adhesive which weakens on exposure to liquid.

Advantageously, the likelihood of a sachet according to the invention failing to rupture when exposed to liquid is minimised. Therefore, embodiments of the invention made from paper and having a high rupture rate may be manufactured cheaply and efficiently.

The adhesive may be a solvent based liquid. The or each sheet of material may be coated with adhesive. Alternatively, the or each sheet of material may be partially coated with adhesive at regions at which the or each sheet is fastened together. The adhesive may be applied at a rate of 1.5 gsm. The adhesive may be applied at a rate of 0.9 gsm. The adhesive may be a lacquer.

The fastening means may additionally comprise a thread.

The or each sheet of material may comprise paper. The basis weight of paper may be 22 gsm. The paper may comprise machine glazed paper. The paper may be perforated with holes of diameter 300 microns to thereby allow liquid to pass through the walls of the sachet. The perforations may be covered with water soluble lacquer to thereby restrict the transfer of liquid across the sheets of material until exposed to sufficient liquid to cause the lacquer to dissolve and expose the perforations.

The quantity of sodium polyacrylate granules may be chosen such that the swelled volume is at least five times the volume of the sachet. The quantity of sodium polyacrylate granules may be chosen such that the swelled volume is at least ten times the volume of the sachet. The quantity of sodium polyacrylate granules may be chosen such that the swelled volume is at least fifty times the volume of the sachet.

The or each sheet of material may define a space within which the sodium polyacrylate granules are contained. The sachet may comprise a single sheet of material that has been folded and fastened together along the peripheral edges of the folded sides. Alternatively, the sachet may comprise two sheets of material fastened together along their respective peripheral edges. The or each sheet of material may be fastened to itself and/or another sheet of material such that the sachet is sealed along the entire length of the peripheral edges. Alternatively, the or each sheet may be fastened to itself and/or another sheet of material such that the sachet is partially sealed along the length of the peripheral edges. Those regions of the peripheral edges that are not sealed together may be sized such that sodium polyacrylate granules cannot exit the sachet when not exposed to liquid.

In order that the invention may be more clearly understood embodiments thereof will now be described, by way of example, with reference to the accompanying drawings of which:
Fig. 1 is a plan view of a sachet according to the invention;
Fig. 2 is a cross sectional side view of the sachet shown in Fig. 1;
Fig. 3 is an enlarged side view of an end of the sachet shown in Fig. 2;
Fig. 4 is a cross sectional side view of the sachet shown in Fig. 2 shortly after being exposed to a liquid;
Fig. 5 is a cross sectional view of the sachet shown in Fig. 4 when the absorbent material has breached the interface between the sides of the sachet;
Fig. 6 is a cross sectional side view of the sachet shown in Fig. 4 when the absorbent material has breached the interface between the sides of the sachet and also breached a side of the sachet; and
Fig. 7 is an enlarged side view of an end of a different embodiment of the sachet shown in Fig. 2;

Referring to the drawings, there is shown a burstable or rupturable sachet 1 for absorbing liquid made from two roughly square sheets of 22 gsm machine glazed bleached kraft paper 3, 5 that have been sealed together along their peripheral edges 7, 9 to form an enclosed volume of space 11. Both sheets of paper 3, 5 are water permeable having holes of diameter 300 microns throughout their respective surfaces in a PI pattern. The two sheets are heat welded together along their four peripheral edges 7, 9 with a layer of water soluble, solvent based lacquer 10 applied at 1.5 gsm and disposed at the interface between the two edges to form a line of seal 20 surrounding the space 11. The sachet 1 is particularly suited to the hospital environment where it is desirable to safely clean up unwanted liquid chemicals, or liquid waste from patients such as blood, urine and vomit and with which, physical contact is undesirable.

A quantity of dry granules of sodium polyacrylate, a super absorbent polymer (SAP) 13 is sealed within the space 11 defined by the sachet 1. Sodium polyacrylate has the ability to absorb and retain 200 to 300 times its mass in water which causes the powdered granules to solidify and form a gel. The quantity of SAP 13 sealed within the sachet 1 is chosen such that, when the sachet is exposed to water containing liquid, a swelled volume of SAP exceeds the volume of the space 11 in which the SAP 13 is contained to thereby cause the sachet 1 to rupture.

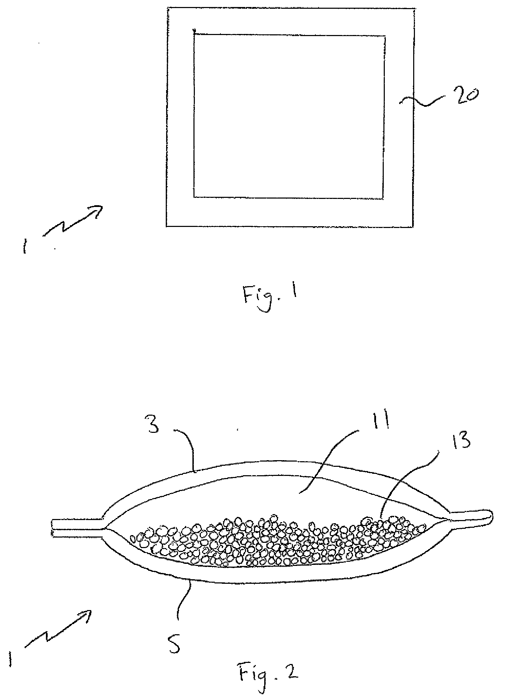

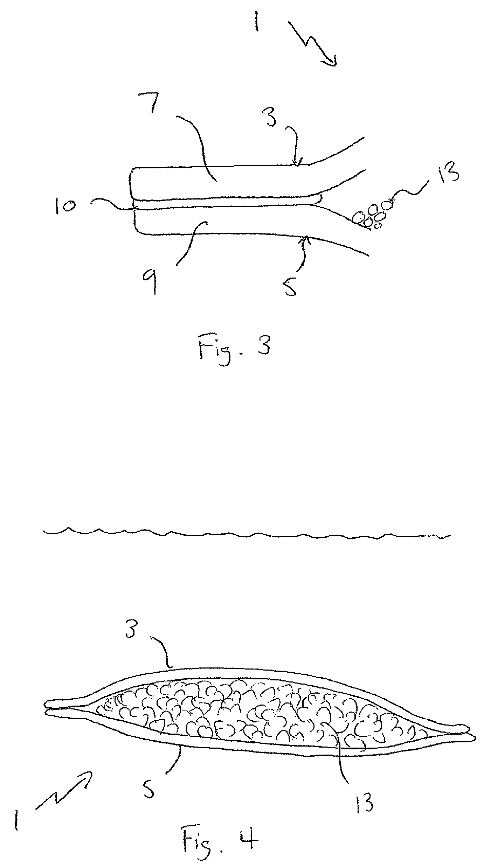

Refering to Fig. 2, the volume of dry granules of SAP 13 is less than that of the space 11 defined by the sachet 1. When the sachet 1 is brought into contact with water containing liquid, the water permeable paper permits the liquid to enter the space 11 and come into direct contact with the SAP 13. As shown in Fig. 4, the exposure of the SAP granules 13 to water results in absorption of the water by the SAP granules 13 which causes them to swell and increase in volume to form a gel like consistency. If sufficient water comes into contact with the SAP granules 13, they swell to the extent that they fill the space 11 defined by the sachet 1 and exert force upon its walls 3, 5.

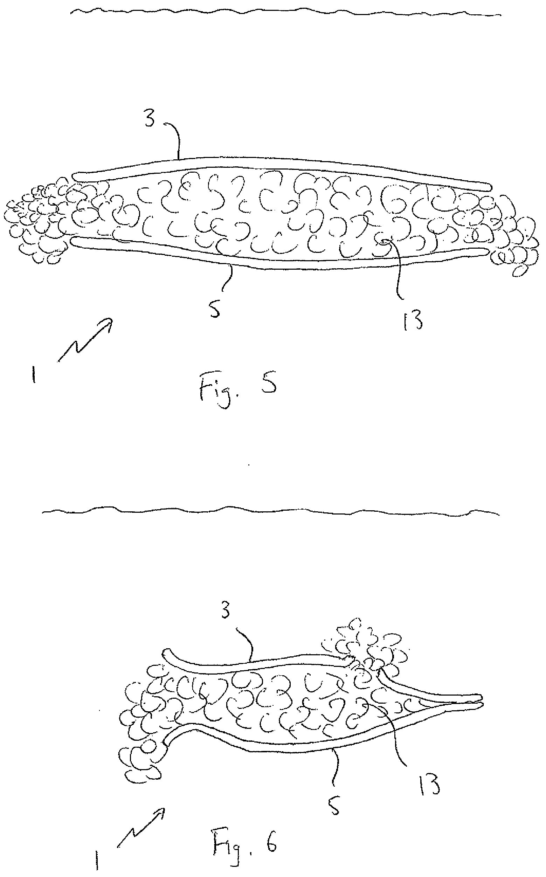

Exposing the sachet 1 to water containing liquid also causes the water soluble lacquer 10 to dissolve which weakens the bond at the interface between the two sheets 3, 5 of the sachet 1. As shown in Fig. 5, the weakening of the bond between the sheets 3, 5 coupled with the increased internal pressure of the sachet 1 resulting from the expansion of the SAP granules causes the seal 20 to break at one or more locations. It can also result in the complete detachment of the two sheets 3, 5 from one another. The breaking of the seal 20 permits the SAP granules 13 to spread beyond the walls 3, 5 of the sachet 1 and become dispersed throughout the liquid where they can continue their absorption and expansion. Thus the act of rupturing the bag and dispersing the SAP 13 through a liquid maximises the SAP's absorbing ability.

In addition to causing a breach at the weakened interface between the sheets 3, 5, as with conventional paper sachets, the expansion of the SAP granules 13 can also result in a rupture at the surface of a sheet of material 3, 5 due to the pressure exerted by the SAP granules 13 overcoming the wet strength of the paper. Thus the combination of a paper sachet with a water soluble lacquer helps to maximise the chances of the sachet 1 bursting and releasing the SAP granules 13 contained within.

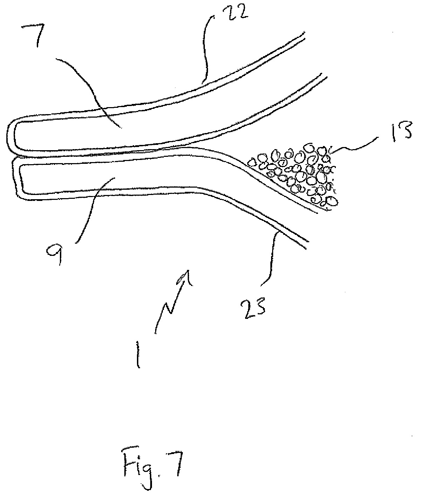

Fig. 7 illustrates a second embodiment in which the inner and outer facing sides of both sheets of the sachet are coated with a 1.5 gsm layer of water soluble lacquer, but in other respects the second embodiment is similar to the first. A sachet 1 with water soluble lacquer 22, 23 coating its sides provides added support and strength to the paper and therefore permits the use of paper with a lower tensile strength than that of the first embodiment. A coated paper of lower tensile strength is more robust that the raw paper and can therefore be run through sachet manufacturing machines without tearing or burning.

When a sachet 1 according to the second embodiment is exposed to water containing liquid, the water soluble lacquer 22, 23 dissolves from the inner and outer surfaces thereby permitting the water to pass through the perforations in the sides of the sachet and come into contact with the SAP granules 13. Since the paper is of a lower tensile strength that that of the first embodiment, when the water soluble lacquer 22, 23 has partially or fully dissolved from the sachet 1, the lower strength paper is left exposed and therefore is more prone to rupture as a result of the rapidly expanding SAP granules 13.

In addition, since the perforations of the sachet 1 are covered by lacquer 22, 23, the SAP granules 13 contained within are not activated until the lacquer is partially or fully dissolved which means that activation of the sachet 1 does not occur until the sachet 1 is exposed to a sufficient quantity of water. Thus accidental activation of a sachet 1 by small quantities of water is minimised.

It is of course to be understood that the above embodiments have been described by way of example only and that many variations are possible without departing from the scope of the invention.

## Claims

1. A sachet (1) defining a volume (11), the sachet comprising a sheet of paper fastened to itself and/or another sheet of paper and containing a quantity of dry granules of sodium polyacrylate (13) capable of absorbently swelling to exceed the volume of the sachet when the sachet is exposed to liquid, wherein the fastening means comprises a water soluble, heat activatable adhesive (10, 22, 23), which weakens on exposure to liquid.

2. A sachet as claimed in any preceding claim, wherein the adhesive is a solvent based liquid.

3. A sachet as claimed in any preceding claim, wherein the sheet material is coated with the adhesive.

4. A sachet as claimed in either of claims 1 or 2, wherein the sheet material is partially coated with the adhesive at regions (7, 9) at which the or each sheet is fastened together.

5. A sachet as claimed in any preceding claim, wherein the adhesive is a lacquer.

6. A sachet as claimed in any preceding claim, wherein the fastening means additionally comprises a thread.

7. A sachet as claimed in any preceding claim, wherein the or each sheet of material comprises machine glazed paper.

8. A sachet as claimed in any preceding claim, wherein the paper is perforated with holes.

9. A sachet as claimed in claim 8, wherein the perforations are covered with water soluble lacquer to restrict the transfer of liquid across the sheets of material until exposed to sufficient liquid to cause the lacquer to dissolve and expose the perforations.

10. A sachet as claimed in any preceding claim, wherein the quantity of dry granules of sodium polyacrylate is chosen such that the swelled volume is at least five times the volume of the sachet.

11. A sachet as claimed in any preceding claim, wherein the sachet comprises a single sheet of material that has been folded and fastened together along the peripheral edges of the folded sides.

12. A sachet as claimed in any of claims 1 to 10, wherein the sachet comprises two sheets of material fastened together along their respective peripheral edges.

13. A sachet as claimed in claim 11 or claim 12, wherein the or each sheet is fastened to itself and/or another sheet of material such that the sachet is sealed along the entire length of the peripheral edges.

14. A sachet as claimed in claim 11 or claim 12, wherein the or each sheet is fastened to itself and/or another sheet of material such that the sachet is partially sealed along the length of the peripheral edges.

15. A sachet as claimed in claim 14, wherein the regions of the peripheral edges that are not sealed together are sized such that the absorbent material cannot exit the sachet when not exposed to liquid.

## Patentansprüche

1. Säckchen (1) definierend ein Volumen (11), wobei das Säckchen ein Blatt Papier aufweist, welches mit sich und/oder an einem anderen Blatt Papier festgemacht ist und eine Menge an trockenen Granulaten aus Natriumpolyacrylat (13) aufweist, welche dazu geeignet sind, absorbierend schwellend zu sein, um das Volumen des Säckchens zu übersteigen, wenn das Säckchen Flüssigkeit ausgesetzt ist, wobei das Befestigungsmittel einen wasserlöslichen, durch Wärme aktivierbaren Klebstoff (10, 22, 23) aufweist, welcher durch die Einwirkung von Flüssigkeit geschwächt wird.

2. Säckchen nach einem der vorangehenden Ansprüche, wobei der Klebstoff eine auf der Basis von Lösungsmittelgebildete Flüssigkeit ist.

3. Säckchen nach einem der vorangehenden Ansprüche, wobei das Blattmaterial mit dem Klebstoff beschichtet ist.

4. Säckchen nach einem der Ansprüche 1 oder 2, wobei das Blattmaterial teilweise mit dem Klebstoff in Regionen (7, 9) beschichtet ist, an welchen das oder jedes Blatt miteinander festgemacht ist.

5. Säckchen nach einem der vorangehenden Ansprüche, wobei der Klebstoff ein Lack ist.

6. Säckchen nach einem der vorangehenden Ansprüche, wobei das Befestigungsmittel zusätzlich einen Faden aufweist.

7. Säckchen nach einem der vorangehenden Ansprüche, wobei das oder jedes Materialblatt maschinengeglänztes Papier aufweist.

8. Säckchen nach einem der vorangehenden Ansprüche, wobei das Papier mit Löchern perforiert ist.

9. Säckchen nach Anspruch 8, wobei die Perforierungen mit wasserlöslichem Lack überzogen sind, um die Übertragung von Flüssigkeit über die Materialblätter solange zu beschränken, bis sie Flüssigkeit in ausreichendem Umfang ausgesetzt sind, um eine Auflösung des Lacks und eine Freilegung der Perforationen zu bewirken.

10. Säckchen nach einem der vorangegangenen Ansprüche, wobei die Menge an trockenen Granulaten aus Natriumpolyacrylat derart ausgewählt ist, dass das angeschwollene Volumen wenigstens das fünffache Volumen des Säckchens ist.

11. Säckchen nach einem der vorangegangenen Ansprüche, wobei das Säckchen ein einzelnes Materialblatt aufweist, welches entlang der peripheren Ränder der gefalteten Seiten zusammen gefaltet und befestigt wurde.

12. Säckchen nach einem der Ansprüche 1 bis 10, wobei das Säckchen zwei Materialblätter aufweist, die entlang ihrer jeweiligen peripheren Ränder aneinander festgemacht sind.

13. Säckchen nach Anspruch 11 oder 12, wobei das oder jedes Blatt mit sich selbst und/oder einem anderen Materialblatt derart festgemacht ist, dass das Säckchen entlang der gesamten Länge der peripheren Ränder verschweißt ist.

14. Säckchen nach Anspruch 11 oder 12, wobei das oder jedes Blatt mit sich selbst und/oder einem anderen Materialblatt derart festgemacht ist, dass das Säckchen teilweise entlang der Länge der peripheren Ränder verschweißt ist.

15. Säckchen nach Anspruch 14, wobei die Bereiche der peripheren Ränder, die nicht miteinander verschweißt sind, derart bemessen sind, dass das absorbierende Material das Säckchen nicht verlassen kann, wenn es keiner Flüssigkeit ausgesetzt ist.

## Revendications

1. Sachet (1) définissant un volume (11), le sachet comprenant une feuille de papier fixée sur elle-même et / ou sur une autre feuille de papier, et contenant une quantité de granules secs de polyacrylate de sodium (13) capables de gonfler par absorption de façon à excéder le volume du sachet lorsque le sachet est exposé à un liquide, dans lequel les moyens de fixation comprennent un adhésif soluble dans l'eau et qui peut être activé par la chaleur (10, 22, 23), qui s'affaiblit au contact du liquide.

2. Sachet selon la revendication 1, dans lequel l'adhésif est un liquide à base de solvant.

3. Sachet selon l'une quelconque des revendications précédentes, dans lequel le matériau de feuille est enduit avec l'adhésif.

4. Sachet selon la revendication 1 ou la revendication 2, dans lequel le matériau de feuille est enduit en partie avec l'adhésif au niveau des régions (7, 9) où est fixée la feuille ou chacune des feuilles.

5. Sachet selon l'une quelconque des revendications précédentes, dans lequel l'adhésif est une laque.

6. Sachet selon l'une quelconque des revendications précédentes, dans lequel les moyens de fixation comprennent en outre un cordon.

7. Sachet selon l'une quelconque des revendications précédentes, dans lequel la feuille ou chacune des feuilles de matériau, comprend un papier frictionné sur machine.

8. Sachet selon l'une quelconque des revendications précédentes, dans lequel le papier est perforé avec des trous.

9. Sachet selon la revendication 8, dans lequel les perforations sont recouvertes par une laque soluble dans l'eau de façon à limiter le transfert du liquide à travers les feuilles de matériau jusqu'à ce qu'elles soient exposées à suffisamment de liquide afin de provoquer une dissolution de la laque et une exposition des perforations.

10. Sachet selon l'une quelconque des revendications précédentes, dans lequel la quantité de granules secs de polyacrylate de sodium, est sélectionnée de telle sorte que le volume gonflé soit au moins cinq fois égal au volume du sachet.

11. Sachet selon l'une quelconque des revendications précédentes, dans lequel le sachet comprend une seule feuille de matériau qui a été pliée et fixée sur elle-même le long des bords périphériques des côtés pliés.

12. Sachet selon l'une quelconque des revendications 1 à 10, dans lequel le sachet comprend deux feuilles de matériau fixées ensemble le long de leurs bords périphériques respectifs.

13. Sachet selon la revendication 11 ou la revendication 12, dans lequel la feuille ou chacune des feuilles est fixée à elle-même et / ou sur une autre feuille de matériau de telle sorte que le sachet soit scellé sur toute la longueur des bords périphériques.

14. Sachet selon la revendication 11 ou la revendication 12, dans lequel la feuille ou chacune des feuilles est fixée à elle-même et / ou sur une autre feuille de matériau de telle sorte que le sachet soit scellé sur partie de la longueur des bords périphériques.

15. Sachet selon la revendication 14, dans lequel les régions des bords périphériques qui ne sont pas scellées ensemble, présentent des dimensions telles que le matériau absorbant ne puisse pas sortir du sachet quand il n'est pas exposé au liquide.
